# EUROPEAN PATENT APPLICATION

(11) **EP 4 834 885 A1**
(43) Date of publication of application: **04.11.2026**
(21) Application number: 25173189.9
(22) Date of filing: 29.04.2025
(51) Int. Cl.: C07D 239/54, A01N 43/54

(54) **HYDROGENATION PROCESS FOR THE PREPARATION OF SUBSTITUTED N-PHENYLURACILS**

(71) Applicant: Bayer Aktiengesellschaft, 51373 Leverkusen (DE)
(72) Inventor: The designation of the inventor has not yet been filed
(74) Representative: BIP Patents

(57) **Abstract**

The present invention relates to substituted N-phenyluracils of formula (I) as well as to processes for the synthesis of such compounds and their use as important intermediates in the synthesis of agrochemical and pharmaceutical active ingredients.

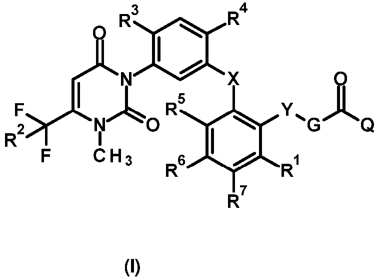

## Description

The present invention relates to substituted N-phenyluracils of formula (I) as well as to processes for the synthesis of such compounds and their use as important intermediates in the synthesis of agrochemical and pharmaceutical active ingredients.

Compounds of formula (I) are not known so far but can be important intermediates in the agrochemical and pharmaceutical production industry. Accordingly, the synthesis of compounds of formula (I) has not been described so far.

Herbicidally active substituted N-phenyluracils are known in the art, *see* WO2024/078906. Due to the various and partly reactive by-groups of such compounds, their synthesis is challenging. It is desirable to adapt the synthesis of such compounds to increase yield and purity at reasonable costs in large quantities.

Furthermore, hydrogenation reactions of certain substituted uracils with noble metal catalysts, such as palladium, are described in the art *(see* WO2011/137088). However, depending on the side groups on such multi-substituted and partly unsaturated structures, it is challenging to perform such a hydrogenation reaction in a selective manner without significant built-up of unwanted side-products. It has been found that, for example, palladium catalysts as used in WO2011/137088 are not sufficiently selective with respect to the uracil structure of compounds of formula (I), i.e. these catalysts hydrogenate also the uracil in an unwanted side reaction. A selective hydrogenation reaction is however required to reduce costs and environmental impact.

At least parts of these problems have been solved by the present invention.

The present invention relates to a hydrogenation reaction for the preparation of compounds of formula (**I**) 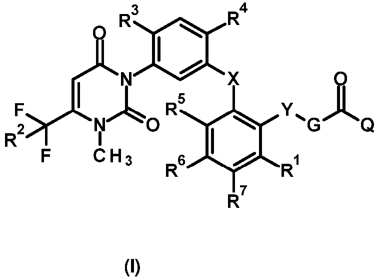
- R¹: is hydrogen, fluorine, chlorine or bromine,
- R²: is fluorine,
- R³: is fluorine,
- R⁴: is NH₂,
- R⁵: is hydrogen, fluorine, chlorine or bromine,
- R⁶: is hydrogen, fluorine, chlorine, bromine or cyano,
- R⁷: is hydrogen, fluorine, chlorine or bromine,
- G: is methylene, (methyl)methylene or (ethyl)methylene,
- X and Y: are independently O (oxygen) or S (sulfur)
and
- Q: is hydroxy or one of the moieties Q-1 to Q-25 specifically mentioned below, where the arrow in the structural formulae in the table below represents a bond of the respective Q group to carbonyl group in the general formula (I),

| | | | | |
|---|---|---|---|---|
| 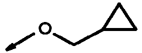 | 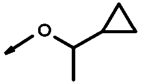 | 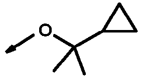 | 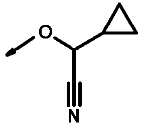 | 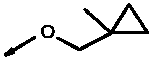 |
| Q-1 | Q-2 | Q-3 | Q-4 | Q-5 |

| | | | | |
|---|---|---|---|---|
| 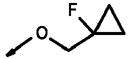 | 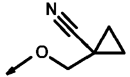 | 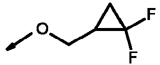 | 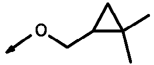 | 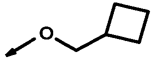 |
| Q-6 | Q-7 | Q-8 | Q-9 | Q-10 |

| | | | | |
|---|---|---|---|---|
| 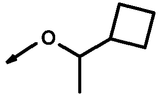 | 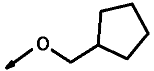 | 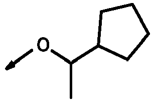 | 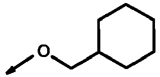 | 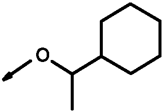 |
| Q-11 | Q-12 | Q-13 | Q-14 | Q-15 |

| | | | | |
|---|---|---|---|---|
| 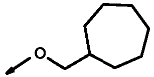 | 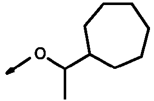 | 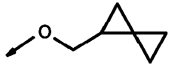 | 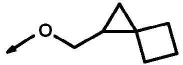 | 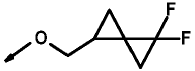 |
| Q-16 | Q-17 | Q-18 | Q-19 | Q-20 |

| | | | | |
|---|---|---|---|---|
| 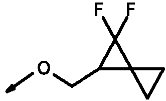 | 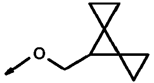 | 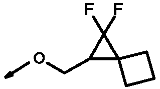 | 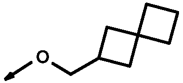 | 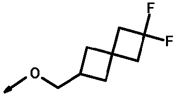 |
| Q-21 | Q-22 | Q-23 | Q-24 | Q-25 |

wherein compounds of formula (**II**) 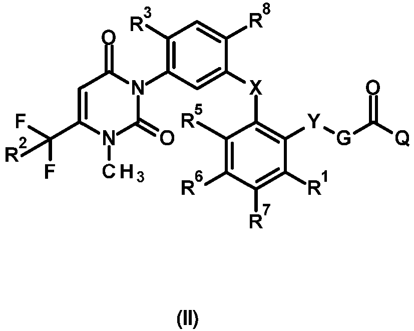 wherein R⁸ is NO₂,
and wherein the definitions of R¹, R², R³, R⁵, R⁶, R⁷, X, Y, G and Q of formula (I) otherwise apply to the compounds of formula **(II**),
are hydrogenated in the presence of H₂ and in the presence of a catalyst.

The hydrogenation of compounds of formula (II) to the compounds of formula (I) may proceed via the intermediates of formula (III), 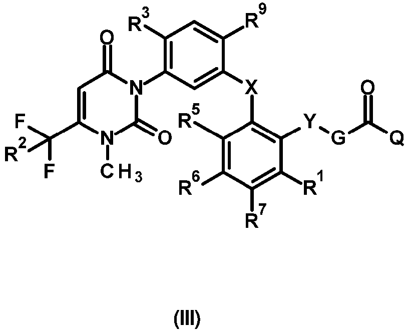 wherein R⁹ is NHOH, and wherein the definitions of R¹, R², R³, R⁵, R⁶, R⁷, X, Y, G and Q of formula (I) otherwise apply to the compounds of formula (III).

Preferably, in compounds of formula (I), (II) and (III),
- R¹: is hydrogen,
- R²: is fluorine,
- R³: is fluorine,
- R⁵: is hydrogen,
- R⁶: is hydrogen, fluorine, chlorine,
- R⁷: is hydrogen,
- G: is methylene, (methyl)methylene,
- X: is O (oxygen) or S (sulfur),
- Y: is O (oxygen)
and
- Q: is hydroxy or one of the moieties Q-1 to Q-25 specifically mentioned above.

Further preferably, in compounds of formula (I), (II) and (III),
- R¹: is hydrogen,
- R²: is fluorine,
- R³: is fluorine,
- R⁵: is hydrogen,
- R⁶: is hydrogen or fluorine,
- R⁷: is hydrogen,
- G: is methylene,
- X: is O (oxygen),
- Y: is O (oxygen)
and
- Q: represents hydroxy or one of the moieties Q-1, Q-2, Q-4, Q-5. Q-6, Q-7, Q-8, Q-9, Q-10, Q-12, Q-13, Q-14, Q-15, Q-16, Q-18 or Q-19.

Even further preferably, in compounds of formula (I), (II) and (III),
- R¹: is hydrogen,
- R²: is fluorine,
- R³: is fluorine,
- R⁵: is hydrogen,
- R⁶: is hydrogen,
- R⁷: is hydrogen,
- G: is methylene,
- X: is O (oxygen),
- Y: is O (oxygen)
and
- Q: is hydroxy or Q-1.

The catalyst preferably comprises platinum on a solid support.

The solid support may comprise carbon, aluminum oxide, calcium carbonate, or silicium dioxide, or mixtures thereof.

Preferably, the solid support is carbon, such as activated carbon.

Preferably, the platinum is loaded onto the solid support in a concentration of between 0.1 and 10 wt.%, based on the total weight of the catalyst.

Further preferably, the platinum is loaded onto the solid support in a concentration of between 0.8 and 5 wt.%, based on the total weight of the catalyst.

The hydrogenation reaction may be performed in the presence of an additional metal loaded on the solid support.

Preferably, the additional metal is selected from molybdenum, vanadium, iron, copper, or mixtures thereof.

Further preferably, the additional metal is selected from molybdenum, vanadium and iron.

The additional metal loading is preferably loaded onto the solid support in a concentration of between 0 and 5 wt.%, further preferably between 0.1 and 2 wt.%, based on the total weight of the catalyst.

The hydrogenation reaction may be performed in an organic solvent. The organic solvent may be selected from alcoholic solvents, such as methanol and ethanol, ethereal solvents, such as tert-butylmethylether, carbonates, such as dimethyl carbonate, or nitrogen containing organic solvents, such as acetonitrile.

Preferably, the organic solvent is acetonitrile.

The hydrogenation reaction may be performed at a temperature of between 20 °C and 80 °C, preferably between 30 °C and 70 °C, further preferably between 40 °C and 60 °C.

The hydrogenation reaction may be performed at a pressure of between 1 and 30 bar hydrogen, preferably between 1 and 5 bar hydrogen.

The hydrogenation reaction may be conducted as a batch hydrogenation wherein compounds of formula (II), a solvent and a catalyst are all filled into the reactor and then the reaction is conducted under hydrogen pressure until completion.

Alternatively, the hydrogenation reaction may be performed as a fed-batch hydrogenation wherein only the solvent and the catalyst are filled into the reactor and a solution of the compounds of formula (II) in a solvent is added slowly to the stirred and heated reactor under hydrogen pressure. The addition time of compounds of formula (II) in a fed-batch reaction may be between 1-16 h, preferably between 3-10 h. Fed-batch hydrogenation allows further reduction of built-up of unwanted side-products.

### Analytical methods

LC-MS were measured with a Waters Acquity IClass and a SQD2 mass spectrometer (ESI)
Column: Zorbax Eclipse Plus C18 50 mm * 2,1 mm, temperature: 55°C
Eluent A: 1 ml formic acid per liter acetonitrile
Eluent B: 0.9 ml formic acid per liter water
Linear gradient, 0.0 to 1.70 min 10% A to 95% A, 1.70 to 2.40 min 95% A, flow 0.85 ml/min.
NMR spectra were measured with a Bruker AV III 600.

### Examples

### Example 1-40:

A solution of 0.5 g (0.9 mmol) of compound 2 (cyclopropylmethyl 2-[2-[4-fluoro-5-[3-methyl-2,6-dioxo-4-(trifluoromethyl)pyrimidin-1-yl]-2-nitro-phenoxy]phenoxy]acetate) in 4.5 g acetonitrile and 20 mg of catalyst of table 1 were added to a test tube containing a magnetic stirring bar. The mixture was stirred under 3 bar hydrogen at 50°C for 16 h. The obtained reaction solution was analyzed by LC-MS yielding the compounds 1, 2, 3 or by-products, such as compound 4, by area % according to table 1.

### Compound 1: cyclopropylmethyl 2-[2-[2-amino-4-fluoro-5-[3-methyl-2,6-dioxo-4-(trifluoromethyl)pyrimidin-1-yl]phenoxy]phenoxy]acetate

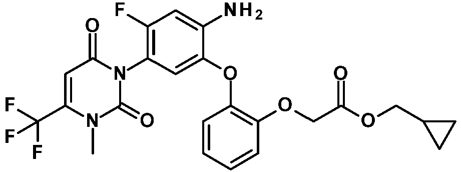

### Compound 2: cyclopropylmethyl 2-[2-[4-fluoro-5-[3-methyl-2,6-dioxo-4-(trifluoromethyl)pyrimidin-1-yl]-2-nitro-phenoxy]phenoxy] acetate

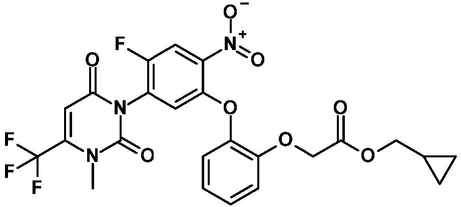

### Compound 3: cyclopropylmethyl 2-[2-[4-fluoro-2-(hydroxyamino)-5-[3-methyl-2,6-dioxo-4-(trifluoromethyl)pyrimidin-1-yl]phenoxy]phenoxy]acetate

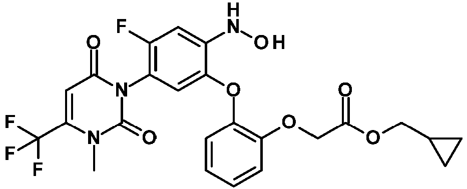

### Compound 4: cyclopropylmethyl 2-[2-[2-amino-4-fluoro-5-[rac-(4R)-3-methyl-2,6-dioxo-4-(trifluoromethyl)hexahydropyrimidin-1-yl]phenoxy]phenoxy]acetate

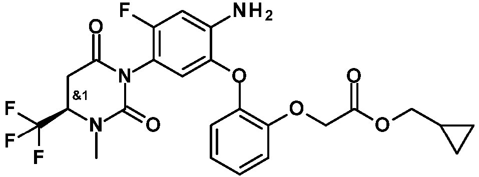

bp: Sum of all other by-products

**Table 1:**

| Example | Catalyst type* | Compound 2 [%] | Compound 1 [%] | Compound 3 [%] | Compound 4 [%] | bp [%] |
|---|---|---|---|---|---|---|
| 1 | 1% Pt+2%V/C | 0 | 100 | 0 | 0 | 0 |
| 2 | 1% Pt+2% V/C | 0 | 99 | 0 | 0 | 1 |
| 3 | 1% Pt+2%V/C | 0 | 97 | 2 | 0 | 1 |
| 4 | 0,8% Pt + 0,6% Mo/C | 0 | 96 | 2 | 0 | 2 |
| 5 | 1,5% Pt+0,1% Fe/C | 0 | 96 | 4 | 0 | 0 |
| 6 | 0,8% Pt +0,3% Mo/C | 0 | 95 | 0 | 5 | 0 |
| 7 | 2% Pt+1%V/C | 0 | 93 | 0 | 7 | 0 |
| 8 | 5% Pt+0,9% Fe/C | 0 | 93 | 2 | 2 | 3 |
| 9 | 2% Pt+1%V/C | 0 | 89 | 2 | 9 | 0 |
| 10 | 1% Pt +0,1% Cu/C | 0 | 52 | 42 | 0 | 6 |
| 11 | 1% *Pt*/*C* | 0 | 52 | 40 | 2 | 6 |
| 12 | 1% Pt/C | 0 | 50 | 45 | 1 | 4 |
| 13 | 1% Pt/C | 4 | 48 | 46 | 0 | 2 |
| 14 | 1% Pt/C | 0 | 46 | 51 | 0 | 3 |
| 15 | 5% Pt(S)/C | 0 | 45 | 22 | 24 | 9 |
| 16 | 5% Pt/C | 0 | 44 | 16 | 31 | 9 |
| 17 | 1,5% Pt/C | 0 | 44 | 38 | 0 | 18 |
| 18 | 1% Pt/C | 0 | 44 | 52 | 0 | 4 |
| 19 | 5% Pt/Al2O3 | 0 | 44 | 30 | 8 | 18 |
| 20 | 1% Pt/C | 0 | 41 | 55 | 0 | 4 |
| 21 | 1% Pt/C | 0 | 38 | 52 | 0 | 10 |
| 22 | 1% Pt/C +0,1% Cu | 0 | 30 | 61 | 0 | 9 |
| 23 | 2% Pd/SiO2/Al2O3 | 0 | 30 | 28 | 9 | 33 |
| 24 | 5% Pd/CaCO3 | 0 | 21 | 4 | 52 | 23 |
| 25 | 5% Ir/C | 63 | 17 | 19 | 0 | 1 |
| 26 | 0,5% Rh/C | 0 | 7 | 37 | 7 | 49 |
| 27 | 1 % Rh/C | 0 | 4 | 9 | 37 | 50 |
| 28 | 0,8% Pt/C | 0 | 2 | 43 | 1 | 54 |
| 29 | 5% Ru/Al2O3 | 95 | 2 | 3 | 0 | 0 |
| 30 | 5% Pd(S)/C | 7 | 2 | 0 | 91 | 0 |
| 31 | 0,5% Pd/C | 49 | 1 | 37 | 0 | 13 |
| 32 | 20% Pd/C | 6 | 0 | 0 | 94 | 0 |
| 33 | 5% Ru/C | 98 | 0 | 1 | 0 | 1 |
| 34 | 5% Pd/C | 13 | 0 | 0 | 87 | 0 |
| 35 | 5% Pd/Al2O3 | 11 | 0 | 0 | 89 | 0 |
| 36 | 5% Pd/C | 10 | 0 | 0 | 90 | 0 |
| 37 | 5% Pd/BaSO4 | 0 | 0 | 0 | 97 | 3 |
| 38 | 5% Pd/CaCO3 + Pb | 0 | 0 | 2 | 34 | 64 |
| 39 | 3% Pd + 2% Pt/C | 10 | 0 | 0 | 86 | 4 |
| 40 | 10% Pd/C | 10 | 0 | 0 | 90 | 0 |

| | | | | | | |
|---|---|---|---|---|---|---|
| **Notation "x% a+y% b*/*C" refers to metals a and b both loaded onto a solid support of carbon (C) in the indicated weight percentages* | | | | | | |

As can be derived from the examples 1 to 40, highest yield of compound 1 could be obtained in the presence of a catalyst comprising platinum and vanadium, both loaded onto a solid carbon carrier, as well as platinum and molybdenum or iron, both loaded onto a solid carbon carrier. Other catalysts, such as palladium catalysts resulted in high amounts of unwanted by-products, in particular in compound 4, which has a reduced double bond in the uracil moiety.

### Example 41

80 g (137 mmol, 94.6%) of cyclopropylmethyl 2-[2-[4-fluoro-5-[3-methyl-2,6-dioxo-4-(trifluoromethyl)pyrimidin-1-yl]-2-nitro-phenoxy]phenoxy]acetate, 663 g of acetonitrile and 3.2 g of catalyst (0.8% Pt and 0.3% Mo/C, e.g. NanoSelect^{™} PT 200 (BASF), 50% water) were added to a 2000 ml autoclave. The autoclave was purged 3 times with 5 bar argon and the pressure was released. Then 1 bar hydrogen was added, and the autoclave was stirred at 50°C for 2 h. Then the pressure was increased to 3 bar hydrogen and stirring was continued for 14 h at 50°C until hydrogen uptake was completed. The pressure was released, and the mixture was filtered through a Nutsche filter. The filter cake was washed two times with acetonitrile and the solvent was distilled off under reduced pressure yielding 100.3 g of a viscous resin. The content of cyclopropylmethyl 2-[2-[2-amino-4-fluoro-5-[3-methyl-2,6-dioxo-4-(trifluoromethyl)pyrimidin-1-yl]phenoxy]phenoxy]acetate was determined to 71.5% by quantitative NMR giving a yield of 100%.

LC-MS: Rₜ = 1.32 min, m/z = 524.4 Da [M+H]⁺1H-NMR (DMSO-d6, 600 MHz): 0.25-0.28 (2H, m), 0.49-0.53 (2H, m), 1.05-1.13 (1H, m), 3.36 (3H, s), 3.95 (2H, d), 4.85 (2H, s), 5.53 (2H, br), 6.44 (1H, s), 6.65 (1H, d), 6.71 (1H, d), 6.88 (1H, dd), 6.93-6.97 (1H, m), 7.03-7.09 (2H, m) ppm.

### Example 42

100 g (173 mmol, 95.6%) of cyclopropylmethyl 2-[2-[4-fluoro-5-[3-methyl-2,6-dioxo-4-(trifluoromethyl)pyrimidin-1-yl]-2-nitro-phenoxy]phenoxy]acetate, 200 g of acetonitrile and 4 g of catalyst (0.8% Pt and 0.3% Mo/C, 50% water) were added to a 600 ml autoclave. The autoclave was purged 3 times with 5 bar argon and the pressure was released. Then 3 bar hydrogen were added, and the autoclave was stirred at 50°C for 16 h. The pressure was released, and the mixture was filtered through a Nutsche filter. The filter cake was washed two times with acetonitrile and the solvent was distilled off under reduced pressure yielding 97.5 g of a viscous resin. The content of cyclopropylmethyl 2-[2-[2-amino-4-fluoro-5-[3-methyl-2,6-dioxo-4-(trifluoromethyl)pyrimidin- 1-yl]phenoxy]phenoxy]acetate was determined to 90.2% by quantitative NMR giving a yield of 97.2%.

1H-NMR (DMSO-d6, 600 MHz): 0.25-0.28 (2H, m), 0.49-0.53 (2H, m), 1.05-1.13 (1H, m), 3.36 (3H, s), 3.95 (2H, d), 4.85 (2H, s), 5.53 (2H, br), 6.44 (1H, s), 6.65 (1H, d), 6.71 (1H, d), 6.88 (1H, dd), 6.93-6.97 (1H, m), 7.03-7.09 (2H, m) ppm.

### Example 43

10 g (17.7 mmol, 98%) of cyclopropylmethyl 2-[2-[4-fluoro-5-[3-methyl-2,6-dioxo-4-(trifluoromethyl)pyrimidin-1-yl]-2-nitro-phenoxy]phenoxy]acetate, 20 g of acetonitrile and 0.4 g of catalyst (0.8% Pt and 0.3% Mo/C, 50% water) were added to a 100 ml autoclave. The autoclave was purged 3 times with 5 bar argon and the pressure was released. Then 5 bar hydrogen were added, and the autoclave was stirred at 50°C for 16 h. The pressure was released, and the mixture was filtered through a Nutsche filter. The filter cake was washed two times with acetonitrile and the content of cyclopropylmethyl 2-[2-[2-amino-4-fluoro-5-[3-methyl-2,6-dioxo-4-(trifluoromethyl)pyrimidin-1-yl]phenoxy]phenoxy]acetate was determined by quantitative NMR giving a yield of 98.8%.

### Example 44

10 g (17.7 mmol, 97.8%) of cyclopropylmethyl 2-[2-[4-fluoro-5-[3-methyl-2,6-dioxo-4-(trifluoromethyl)pyrimidin-1-yl]-2-nitro-phenoxy]phenoxy]acetate, 20 g of acetonitrile and 0.4 g of catalyst (1% Pt and 2% V/C, 55% water) were added to a 100 ml autoclave. The autoclave was purged 3 times with 5 bar argon and the pressure was released. Then 3 bar hydrogen were added, and the autoclave was stirred at 50°C for 16 h. The pressure was released, and the mixture was filtered through a Nutsche filter. The filter cake was washed two times with acetonitrile and the content of cyclopropylmethyl 2-[2-[2-amino-4-fluoro-5-[3-methyl-2,6-dioxo-4-(trifluoromethyl)pyrimidin-1-yl]phenoxy]phenoxy]acetate was determined by quantitative NMR giving a yield of 97.4%.

### Example 45

250 g solution of cyclopropylmethyl 2-[2-[4-fluoro-5-[3-methyl-2,6-dioxo-4-(trifluoromethyl)pyrimidin-1-yl]-2-nitro-phenoxy]phenoxy]acetate (44.8 mmol, 9.9%) in acetonitrile and 1 g of catalyst (1% Pt and 0.9% Fe/C, 50% water) were added to a 1000 ml autoclave. The autoclave was purged 3 times with 3 bar argon and the pressure was released. Then 3 bar hydrogen were added, and the autoclave was stirred at 50°C for 7 h. The pressure was released, and the mixture was filtered through a Nutsche filter. The filter cake was washed two times with acetonitrile and the solvent was distilled off under reduced pressure yielding 24.99 g of a foam. The content of cyclopropylmethyl 2-[2-[2-amino-4-fluoro-5-[3-methyl-2,6-dioxo-4-(trifluoromethyl)pyrimidin-1-yl]phenoxy]phenoxy] was determined to 88% by quantitative NMR giving a yield of 93.6%.

### Example 46

85.84 g solution of cyclopropylmethyl 2-[2-[4-fluoro-5-[3-methyl-2,6-dioxo-4-(trifluoromethyl)pyrimidin-1-yl]-2-nitro-phenoxy]phenoxy]acetate (45.2 mmol, 29.1%) in acetonitrile, 164 g acetonitrile and 2 g of catalyst (0.8% Pt and 0.3% Mo/C, 50% water) were added to a 1000 ml autoclave. The autoclave was purged 3 times with 3 bar argon and the pressure was released. Then 3 bar hydrogen were added, and the autoclave was stirred at 50°C for 8 h. The pressure was released, and the mixture was filtered through a Nutsche filter. The filter cake was washed two times with acetonitrile and the solvent was distilled off under reduced pressure yielding 26.4 g of a foam. The content of cyclopropylmethyl 2-[2-[2-amino-4-fluoro-5-[3-methyl-2,6-dioxo-4-(trifluoromethyl)pyrimidin-1-yl]phenoxy]phenoxy]acetate was determined to 88,8% by quantitative NMR giving a yield of 99.1%.

### Example 47 (Fed-Batch process)

200 g acetonitrile and 1 g of catalyst (0.8% Pt and 0.3% Mo/C, 50% water) were added to a 1000 ml autoclave. The autoclave was purged 3 times with 3 bar argon and the pressure was released. Then 3 bar hydrogen were added, and the autoclave was stirred at 50°C. A solution of 25 g (44.4 mmol, 98.3%) cyclopropylmethyl 2-[2-[4-fluoro-5-[3-methyl-2,6-dioxo-4-(trifluoromethyl)pyrimidin-1-yl]-2-nitro-phenoxy]phenoxy]acetate in 25 g acetonitrile was pumped into the autoclave in 5 h and stirring was continued for additional 5 h. The pressure was released, and the mixture was filtered through a Nutsche filter. The filter cake was washed two times with acetonitrile and the solvent was distilled off under reduced pressure yielding 23.9 g of a foam. The content of cyclopropylmethyl 2-[2-[2-amino-4-fluoro-5-[3-methyl-2,6-dioxo-4-(trifluoromethyl)pyrimidin-1-yl]phenoxy]phenoxy]acetate was determined to 96.1% by quantitative NMR giving a yield of 98.8%.

### Example 48

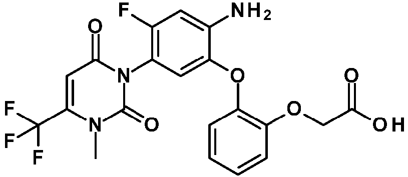

8.43 g (15.5 mmol, 91.8%) of 2-[2-[4-fluoro-5-[3-methyl-2,6-dioxo-4-(trifluoromethyl)pyrimidin-1-yl]-2-nitro-phenoxy]phenoxy]acetic acid, 75 g of acetonitrile and 0.34 g of catalyst (0.8% Pt and 0.3% Mo/C, 50% water) were added to a 300 ml autoclave. The autoclave was purged 3 times with 5 bar argon and the pressure was released. Then 3 bar hydrogen were added, and the autoclave was stirred at 50°C for 16 h. The pressure was released, and the mixture was filtered through a Nutsche filter. The filter cake was washed two times with acetonitrile and the content of 2-[2-[2-amino-4-fluoro-5-[3-methyl-2,6-dioxo-4-(trifluoromethyl)pyrimidin-1-yl]phenoxy]phenoxy]acetic acid was determined by quantitative NMR giving a yield of 98%.
LC-MS: Rₜ = 0.99 min, m/z = 470.4 Da [M+H]⁺
1H-NMR (DMSO-d6, 600 MHz): 3.38 (3H, s), 4.64 (2H, s), 5.53 (2H, br), 6.45 (1H, s), 6.66 (1H, d), 6.77 (1H, d), 6.85-688 (1H, m), 6.89-6.93 (1H, m), 7.01-7.06 (2H, m) ppm.

## Claims

1. Hydrogenation reaction for the preparation of compounds of formula (I) 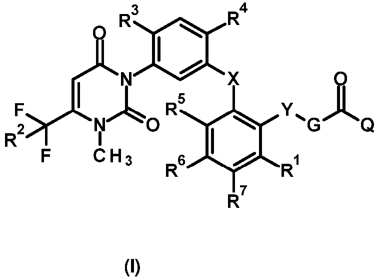
R¹ is hydrogen, fluorine, chlorine or bromine,
R² is fluorine,
R³ is fluorine,
R⁴ is NH₂,
R⁵ is hydrogen, fluorine, chlorine or bromine,
R⁶ is hydrogen, fluorine, chlorine, bromine or cyano,
R⁷ is hydrogen, fluorine, chlorine or bromine,
G is methylene, (methyl)methylene or (ethyl)methylene,
X and Y are independently O (oxygen) or S (sulfur)
and
Q is hydroxy or one of the moieties Q-1 to Q-25 specifically mentioned below, where the arrow in the structural formulae in the table below represents a bond of the respective Q group to carbonyl group in the general formula (I),
| | | | | |
|---|---|---|---|---|
| 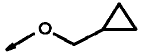 | 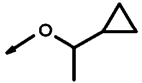 | 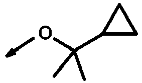 | 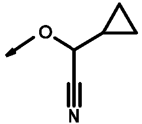 | 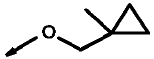 |
| Q-1 | Q-2 | Q-3 | Q-4 | Q-5 |
| | | | | |
|---|---|---|---|---|
| 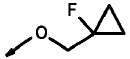 | 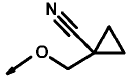 | 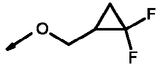 | 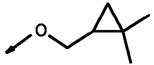 | 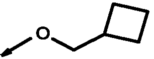 |
| Q-6 | Q-7 | Q-8 | Q-9 | Q-10 |
| | | | | |
|---|---|---|---|---|
| 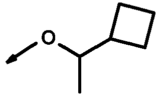 | 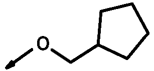 | 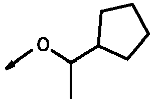 | 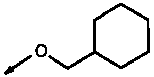 | 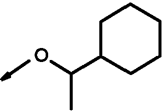 |
| Q-11 | Q-12 | Q-13 | Q-14 | Q-15 |
| | | | | |
|---|---|---|---|---|
| 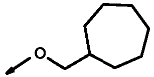 | 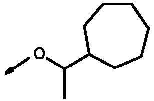 | 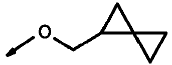 | 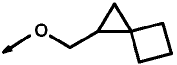 | 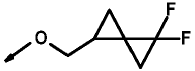 |
| Q-16 | Q-17 | Q-18 | Q-19 | Q-20 |
| | | | | |
|---|---|---|---|---|
| 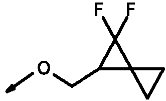 | 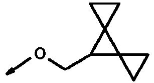 | 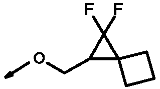 | 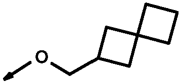 | 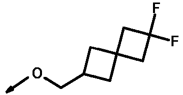 |
| Q-21 | Q-22 | Q-23 | Q-24 | Q-25 |
wherein compounds of formula (II) 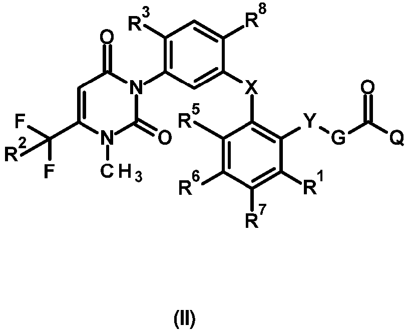 wherein R⁸ is NO₂,
are hydrogenated in the presence of H₂ and in the presence of a catalyst.

2. Hydrogenation reaction according to claim 1, wherein
R¹ is hydrogen,
R² is fluorine,
R³ is fluorine,
R⁵ is hydrogen,
R⁶ is hydrogen, fluorine, chlorine,
R⁷ is hydrogen,
G is methylene, (methyl)methylene,
X is O (oxygen) or S (sulfur),
Y is O (oxygen)
and
Q is hydroxy or one of the moieties Q-1 to Q-25 specifically mentioned above.

3. Hydrogenation reaction according to claim 1, wherein
R¹ is hydrogen,
R² is fluorine,
R³ is fluorine,
R⁵ is hydrogen,
R⁶ is hydrogen or fluorine,
R⁷ is hydrogen,
G is methylene,
X is O (oxygen),
Y is O (oxygen)
and
Q represents hydroxy or one of the moieties Q-1, Q-2, Q-4, Q-5. Q-6, Q-7, Q-8, Q-9, Q-10, Q-12, Q-13, Q-14, Q-15, Q-16, Q-18 or Q-19.

4. Hydrogenation reaction according to claim 1, wherein
R¹ is hydrogen,
R² is fluorine,
R³ is fluorine,
R⁵ is hydrogen,
R⁶ is hydrogen,
R⁷ is hydrogen,
G is methylene,
X is O (oxygen),
Y is O (oxygen)
and
Q is hydroxy or Q-1.

5. Hydrogenation reaction according to any one of the preceding claims, wherein the catalyst comprises platinum on a solid support.

6. Hydrogenation reaction according to claim 5, wherein the solid support comprises carbon, aluminum oxide, calcium carbonate, or silicium hydroxide, or mixtures thereof; preferably wherein the solid support is carbon.

7. Hydrogenation reaction according to any one of claims 5 and 6, wherein the platinum is loaded onto the solid support in a concentration of between 0.1 and 10 wt.%, based on the total weight of the catalyst, preferably in a concentration of between 0.8 and 5 wt.%, based on the total weight of the catalyst.

8. Hydrogenation reaction according to any one of claims 5 to 7, wherein the hydrogenation reaction is performed in the presence of an additional metal loaded on the solid support.

9. Hydrogenation reaction according to claim 8, wherein the additional metal is selected from molybdenum, vanadium, iron, copper, or mixtures thereof, preferably wherein the additional metal is selected from molybdenum, vanadium and iron.

10. Hydrogenation reaction according to any one of claims 8 and 9, wherein the additional metal is loaded onto the solid support in a concentration of between 0 and 5 wt.%, based on the total weight of the catalyst, preferably in a concentration of between 0.1 and 2 wt.%, based on the total weight of the catalyst.

11. Hydrogenation reaction according to any one of the preceding claims, wherein the reaction is performed in an organic solvent, preferably selected from alcoholic solvents, such as methanol and ethanol, ethereal solvents, such as tert-butylmethylether carbonates, such as dimethyl carbonate, or nitrogen containing organic solvents, such as acetonitrile, even further preferably wherein the organic solvent is acetonitrile.

12. Hydrogenation reaction according to any one of the preceding claims, wherein the reaction is performed at a temperature of between 20 °C and 80 °C, preferably between 30 °C and 70 °C, further preferably between 40 °C and 60 °C; and/or wherein the reaction is performed at a pressure of between 1 and 30 bar hydrogen, preferably between 1 and 5 bar hydrogen.

13. Hydrogenation reaction according to any one of the preceding claims, wherein the reaction is performed as a fed-batch hydrogenation reaction.

14. Compounds of formula (I), 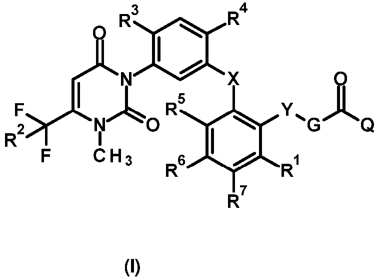 wherein
R¹ is hydrogen, fluorine, chlorine or bromine,
R² is fluorine,
R³ is fluorine,
R⁴ is NH₂,
R⁵ is hydrogen, fluorine, chlorine or bromine,
R⁶ is hydrogen, fluorine, chlorine, bromine or cyano,
R⁷ is hydrogen, fluorine, chlorine or bromine,
G is methylene, (methyl)methylene or (ethyl)methylene,
X and Y are independently O (oxygen) or S (sulfur),
and
Q is hydroxy or one of the moieties Q-1 to Q-25 specifically mentioned below, where the arrow in the structural formulae in the table below represents a bond of the respective Q group to carbonyl group in the general formula (I),
| | | | | |
|---|---|---|---|---|
| 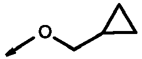 | 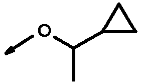 | 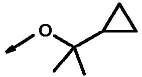 | 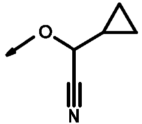 | 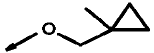 |
| Q-1 | Q-2 | Q-3 | Q-4 | Q-5 |
| | | | | |
|---|---|---|---|---|
| 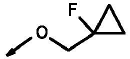 | 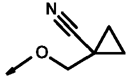 | 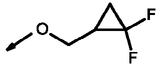 | 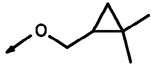 | 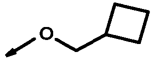 |
| Q-6 | Q-7 | Q-8 | Q-9 | Q-10 |
| | | | | |
|---|---|---|---|---|
| 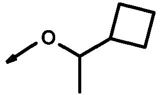 | 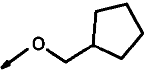 | 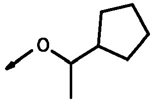 | 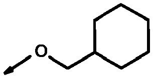 | 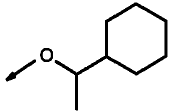 |
| Q-11 | Q-12 | Q-13 | Q-14 | Q-15 |
| | | | | |
|---|---|---|---|---|
| 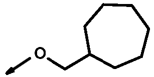 | 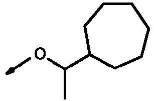 | 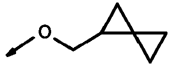 | 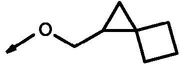 | 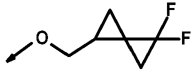 |
| Q-16 | Q-17 | Q-18 | Q-19 | Q-20 |
| | | | | |
|---|---|---|---|---|
| 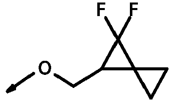 | 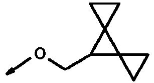 | 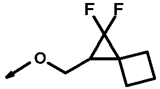 | 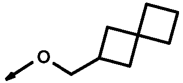 | 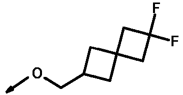 |
| Q-21 | Q-22 | Q-23 | Q-24 | Q-25. |

15. Compounds of formula (III) 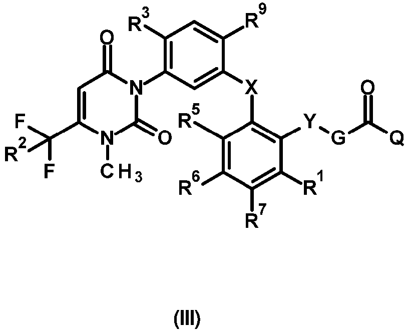 wherein
R¹ is hydrogen, fluorine, chlorine or bromine,
R² is fluorine,
R³ is fluorine,
R⁹ is NHOH,
R⁵ is hydrogen, fluorine, chlorine or bromine,
R⁶ is hydrogen, fluorine, chlorine, bromine or cyano,
R⁷ is hydrogen, fluorine, chlorine or bromine,
G is methylene, (methyl)methylene or (ethyl)methylene,
X and Y are independently O (oxygen) or S (sulfur)
and
Q is hydroxy or one of the moieties Q-1 to Q-25 specifically mentioned below, where the arrow in the structural formulae in the table below represents a bond of the respective Q group to carbonyl group in the general formula (I),
| | | | | |
|---|---|---|---|---|
| 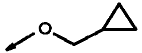 | 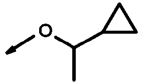 | 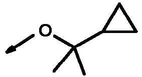 | 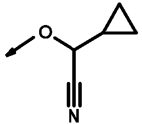 | 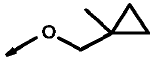 |
| Q-1 | Q-2 | Q-3 | Q-4 | Q-5 |
| | | | | |
|---|---|---|---|---|
| 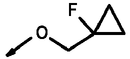 | 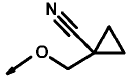 | 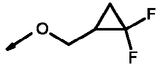 | 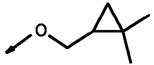 | 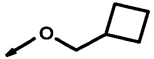 |
| Q-6 | Q-7 | Q-8 | Q-9 | Q-10 |
| | | | | |
|---|---|---|---|---|
| 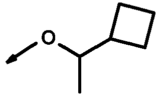 | 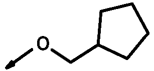 | 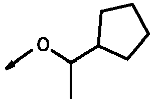 | 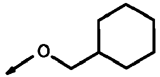 | 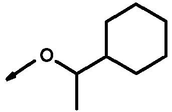 |
| Q-11 | Q-12 | Q-13 | Q-14 | Q-15 |
| | | | | |
|---|---|---|---|---|
| 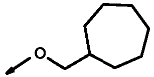 | 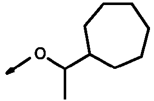 | 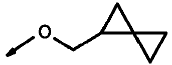 | 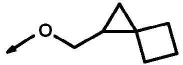 | 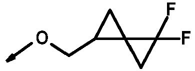 |
| Q-16 | Q-17 | Q-18 | Q-19 | Q-20 |
| | | | | |
|---|---|---|---|---|
| 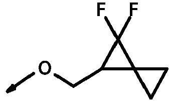 | 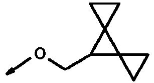 | 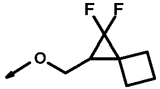 | 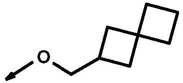 | 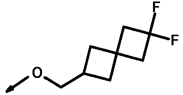 |
| Q-21 | Q-22 | Q-23 | Q-24 | Q-25. |
